# EUROPEAN PATENT SPECIFICATION

(11) **EP 1 935 349 B1**
(45) Date of publication and mention of the grant of the patent: **07.12.2011**
(21) Application number: 07255019.7
(22) Date of filing: 21.12.2007
(51) Int. Cl.: A61B 17/00

(54) **Retractable separating systems**
Exponierbare Trennungssysteme
Systèmes de séparation rétractable

(30) Priority: 22.12.2006 US 615005; 22.12.2006 US 615006
(43) Date of publication of application: 25.06.2008
(73) Proprietor: THE SPECTRANETICS CORPORATION, Colorado Springs Colorado 80907 (US)
(72) Inventor: Taylor, Kevin D., Colorado Springs, CO 80908 (US)
(74) Representative: Calderbank, Thomas Roger

(56) References cited:
- US-A- 6 033 402
- US-A- 6 126 654
- US-B1- 6 884 240

## Description

This application is related to U.S. Patent Application No: 11/615,005 filed December 22, 2006 (titled Tissue Separating Systems and Methods) published as US-A-2003/154296.

The present application relates generally to systems and methods for separating an object from tissue in a patient, and more specifically, to techniques for removing pacing leads from a patient

Cardiac pacing systems typically include a pacemaker and a pacing lead, which are placed inside the body of a patient. The pacemaker includes a power source and circuitry configured to send timed electrical pulses to the pacing lead. The pacing lead carries the electrical pulse to the heart to initiate a heartbeat, and transmits information about the heart's electrical activity to the pacemaker. The pacing lead can include a fixation mechanism that holds the lead to the cardiac tissue. In some cases, a pacing lead is inserted through a vein and guided into a heart chamber where it is attached with the heart. In other instances, a pacing lead is attached to the outside of the heart A common problem associated with pacing leads is the development of scar tissue or adhesions where the pacing lead contacts the patient's body tissue. Patient tissue can become attached with the pacing lead, and thus removal or extraction of the pacing lead may present complications

Current pacing lead extraction techniques include mechanical traction, mechanical devices, and laser devices. Mechanical traction is often accomplished by inserting a locking stylet into the lead and pulling to remove it. In some cases, for example where mechanical traction is ineffective, dilating telescopic sheaths can be used to strip away the scar tissue adhering the lead to the body. Unfortunately, metal sheaths that are currently used to strip scar tissue from implanted leads often cannot traverse the tortuous lead path, and in many instances can only be used in proximal locations. Currently used plastic sheaths may be able to access certain distal lead locations, but often suffer from poor torque properties, low radiopacity, and ineffective penetration into hard tissue because they have soft tips that deform when in contact with the hard tissue. Dilation techniques often involve pushing tissue away from the lead when the sheath is pushed longitudinally along the lead. However, longitudinal forces can be easily lost during the procedure by tortuousity of curvature in the lead and by friction encountered within the anatomy or over the pacing lead. Longitudinal forces also may require heavy counter traction on the lead - that can result in pacing lead breakage. Some mechanical sheaths have proposed trigger mechanisms for extending a blade from a sheath. At least some of these devices, however, involve complicated activation mechanisms and may not be well suited for negotiating the tortuous paths present in certain vascular or physiological environments. Laser devices typically employ laser energy to cut the scar tissue away from the lead thus allowing for removal. Although effective in some circumstances for removing chronic implanted pacing leads, many laser systems can be expensive and unaffordable to many treatment centers.

What is needed are improved devices and methods for extracting pacing leads as well as other objects. These techniques can provide effective alternatives to currently used dilating lead extraction sheaths and laser systems.

US-A-6126654 discloses an elongated sheath catheter which has a handle at its proximal end and a controllably deflectable end portion at its distal end. A distal out jacket portion is connected to a distal tip and a pressure plate mounted in the distal tip which is movable between so as to move a piercing means between a retracted and a protracted portion.

According to the present invention there is provided a separating system for detaching a pacing lead from a patient, comprising:
a sheath having a distal end; and
a separating assembly coupled with the distal end of the sheath, the separating assembly comprising a tip and a separator, the separator having a separating mechanism;
wherein the sheath is fixed with either the tip or the separator, the separating mechanism comprising a circular distal separating edge, the tip comprising a blunt distal end, and each of the sheath, the tip, and the separator are being configured for placement over the pacing lead, the tip and the separator being configured for relative rotational movement;
wherein:
   the separating assembly is adapted to switch upon the relative rotational movement of the tip and the separator between a deployed configuration where the circular distal separating edge of the separating mechanism is exposed and an undeployed configuration where the circular distal separating edge of the separating mechanism is unexposed.

Advantageously, embodiments of the present invention encompass cutting, stripping, and dilating device having designs unique in their simplicity. An exemplary separating system involves a minimum number of parts, for example a retractable assembly and an inexpensive low profile flexible sheath that easily tracks over a pacing lead. Mechanical sheath and retractable assembly embodiments can be safely deployed within the vascular system of a patient. For example, a torqueable and flexible polymer sheath can be coupled with a 2-piece tip section that houses a cutting, stripping, or dilating element. The element can be deployed or exposed to separate tissue by rotating the sheath in one direction. Conversely, the element can be retracted back into the tip by rotating the sheath in the other direction. The penetration action of the sheath can be accomplished by rotation of the sheath to minimize force on the lead and vascular system.

For a fuller understanding of the nature and advantages of the present invention, reference should be had to the ensuing detailed description taken in conjunction with the accompanying drawings.

### IN THE DRAWINGS

FIG. 1 shows a separating system according to embodiments of the present invention.

FIG. 2 shows a separating assembly according to embodiments of the present invention.

FIGS. 3A-3E show a separator according to embodiments of the present invention.

FIGS. 4A-4D show a tip according to embodiments of the present invention.

FIG. 5A-5D show separating assemblies according to embodiments of the present invention.

FIGS. 6A-6C illustrate a process for separating a tissue with a separating system, according to embodiments of the present invention.

FIGS. 7A and 7B illustrate a separating assembly according to embodiments of the present invention.

FIGS. 8A-8C depict a separating assembly according to embodiments of the present invention.

FIGS. 9A and 9B depict a separating assembly according to embodiments of the present invention.

FIGS. 10A and 10B depict a separating assembly according to embodiments of the present invention.

Embodiments of the present invention provide a mechanical sheath and separating tip that can be safely deployed within the vascular system of a patient A separating system can include, for example, a flexible sheath coupled with a separating assembly, which includes a separator and a tip. The separator can be advanced distally beyond the tip by rotating the sheath in one direction. Conversely, the separator can be retracted back into the tip by rotating the sheath in the other direction. The penetration action of the sheath can be accomplished by rotation of the sheath to minimize force on the lead and vascular system.

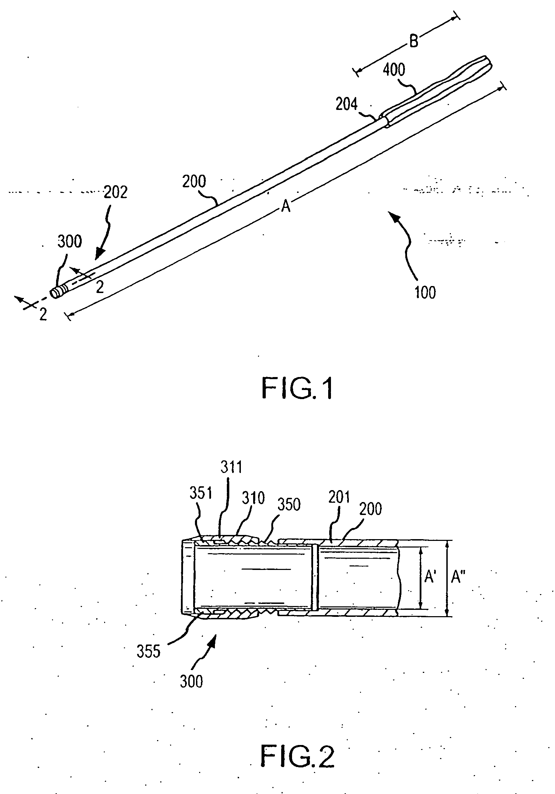

Turning now to the drawings, FIG. 1 shows a separating system or tool 100 according to one embodiment of the present invention. Separating or cutting system 100 includes a sheath 200, a separating assembly 300, and a handle 400. In some embodiments, sheath 200 includes or is manufactured from a flexible polymer such as Pebax^{®} or Teflon^{®}, and may also include a stainless steel braid reinforcement. Sheath 200 may include a distal end 202 coupled with separating assembly 300, and a proximal end 204 coupled with handle 400. An operator can use handle 400 to facilitate rotation of sheath 200 and components of separating assembly 300. In some cases, separating system 100 can have a length A within a range from about 25 cm to about 75 cm. Optionally, separating system 100 can a length A that is about 50 cm. In some cases, handle 400 can have a length B within a range from about 4 cm to about 15 cm. Optionally, handle 400 can a length B that is about 8 cm. Exemplary sheath or shaft 200 configurations are further described in previously incorporated U.S. Patent Application No. 11/615,005, filed December 22, 2006 (Tissue Separating Systems and Methods).

As depicted in FIG. 2, separating assembly 300 can include a separator 310 in operative association with a tip 350. For example, separator 310 and tip 350 can be coupled via a threaded connection. Some of the embodiments described herein refer to cutting elements, cutting assemblies, cutters, and the like, which often include items for cutting tissue, however it is understood that these cutting features can be replaced with or referred to as stripping or dilating elements, stripping or dilating assemblies, or strippers or dilators_{.} Stripping features can include items for stripping tissue from pacing leads and other objects within the body of a patient. Relatedly, dilating features can include items for dilating tissue surrounding or near pacing leads and other objects within the body of a patient. Cutting features or procedures can be used or referred to interchangeably with stripping features or procedures, and with dilating features or procedures. Methods that include stripping or dilating tissue may or may not include cutting tissue. In some embodiments, cutting, stripping, or dilating elements or procedures, or any combinations thereof, may be referred to as separating elements or procedures. For example, a separator may refer to or encompass a cutter, stripper, or a dilator, or any combination thereof.

In some embodiments, sheath 200 is integral with tip 350, such that tip 350 represents a distal end of sheath 200. In some embodiments, a distal end of sheath 200 is fixed with tip 350. A separating assembly may include a marker or other detectable feature that can be imaged. For example, a separating system may include a tip marker 351, a separator marker 311, or a sheath marker 201, or any combination thereof. Such markers may include a radiopaque or other imageable material to allow an operator to determine the relative positional relationships of the separating system components. In some cases, separating system components can be constructed of radiopaque material or alternatively plated with a thin coat of highly radiopaque material such as gold. In one embodiment, a tip marker includes a radiopaque material that allows the operator to distinguish when the separator is extended under fluoroscopy. When placed within a vessel or lumen of a patient, separating assembly 300 is typically disposed in an undeployed configuration, whereby a distal end of separator 310 is flush with or proximal to a distal end of tip 350, or otherwise undeployed or unexposed. In some embodiments, an undeployed configuration can refer to a separating assembly having an unexposed separator blade or a non-separating profile. Once the assembly 300 is positioned at or near a tissue which an operator wishes to separate, the operator can maneuver the assembly 300 so that an external surface of separator 310 frictionally contacts a tissue surface of the patient. The operator can then rotate shaft 200 so that tip 350 rotates relative to separator 310, thus extending a portion of separator 310 distally beyond tip 350. The separating assembly 300 is thus in a deployed configuration and ready for separating the desired tissue. In some embodiments, a deployed configuration can refer to a separating assembly having an exposed separator blade or separating means or mechanism, or presenting a separating profile. In some embodiments, sheath 200 has an inner diameter A' within a range from about 2.29 mm and to about 5.08 mm (about 0.090 to about 0.200 inches) and an outer diameter A" within a range from about 3.30 mm to about 6.35 mm (0.130 to about 0.250 inches). Similarly, handle 400 can have an inner diameter within a range from about 3.30 mm to about 6.35 mm (about 0.130 to about 0.250 inches) and an outer diameter within a range from about 6.35 mm to about 19.05 mm (about 0.250 to about 0.750 inches).

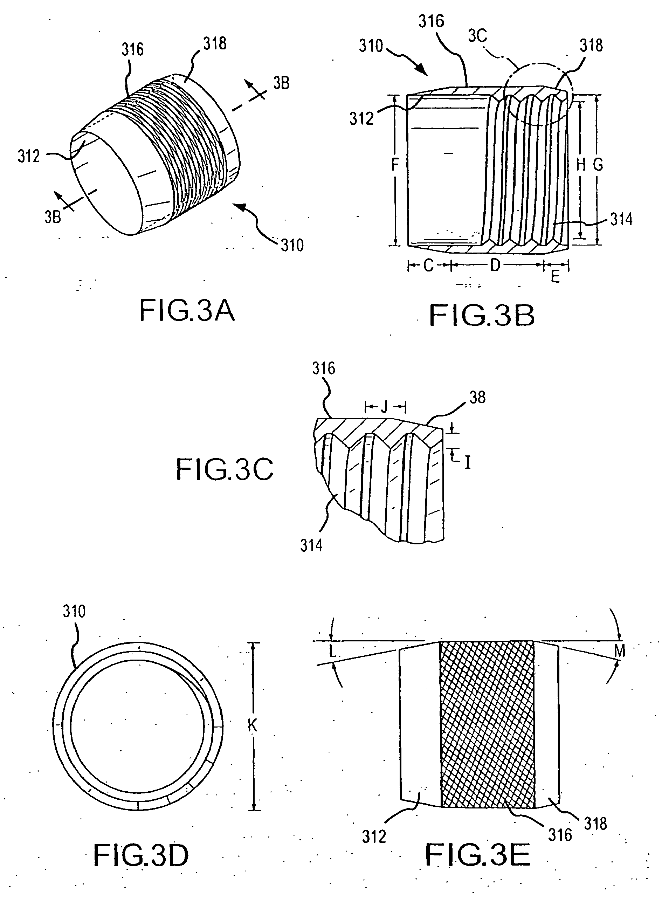

Various features of separator 310 are depicted in Figs. 3A-3E. For example, as illustrated in Fig. 3A, separator 310 can include a distal separating edge 312, a contact region 316, and a proximal edge 318. Contact region 316 may be disposed at any suitable location on separator 310. For example, contact region 316 can be located at a proximal portion of separator 310, a medial portion of separator 310, or at a distal portion, edge, or face of separator 310. In some cases, as shown in Fig. 3B, distal separating edge 312 can have a length C within a range from about 0.25 mm to about 2.50 mm (about 0.01 inch to about 0.10 inch). Optionally, distal separating edge 312 can have a length C of about 1.27 mm (about 0.05 inch). In some embodiments, exterior contact region 316 can have a length D within a range from about 1.52 mm to about 4.06 mm (about 0.06 inch to about 0.16 inch). Optionally, exterior contact region 316 can have a length D of about 2.79 mm (about 0.11 inch). In some cases, proximal edge 318 can have a length E within a range of about 0.25 mm to about 1.27 mm (about 0.01 inch to about 0.05 inch). Optionally, proximal edge 318 can have a length E of about 0.76 mm (about 0.03 inch). In some cases, distal separating edge 312 can have an interior diameter F within a range of about 2.51 mm to about 7.59 mm (about 0.099 inch to about 0.299 inch). Optionally, distal separating edge 312 can have an interior diameter F of about 5.05 mm (about 0.199 inch). In some embodiments, an internal threaded section 314 can have a major thread diameter G within a range from about 2.54 mm to about 7.62 mm (about 0.1 inch to about 0.3 inch). Optionally, major thread diameter G can be about 5.08 mm (about 0.20 inch). In some embodiments, threaded section 314 can have a minor thread diameter H within a range from about 2.03 mm to about 7.11 mm (about 0.08 inch to about 0.28 inch). Optionally, minor thread diameter H can be about 4.57 mm (about 0.18 inch). As illustrated in Fig. 3C, threaded section 314 can define a thread depth I and a thread pitch J. In some cases, thread depth I can be within a range from about 0.13 mm to about 0.38 mm (about 0.005 inch to about 0.015 inch). Optionally, thread depth I can be about 0.25 mm (about 0.010 inch). In some cases, thread pitch J can be within a range from about 0.25 mm to about 0.76 mm (about 0.010 inch to about 0.030 inch). Optionally, thread pitch J can be about 0.51 mm (about 0.020 inch). In some cases, threaded section 314 can have a thread count within a range from about 2.54 threads per mm to about 15.24 threads per mm (about 10 threads per inch to about 60 threads per inch). Optionally, threaded section 314 can have a thread count of about 10 threads per mm (about 40 threads per inch). As depicted in Fig. 3D, cutter 310 can have an outer diameter K within a range from about 3.05mm to about 8.13 mm (about 0.12 inch to about 0.32 inch). Optionally, outer diameter K can be about 5.59 mm (about 0.22 inch). As shown in Fig. 3E, distal cutting edge 312 and contact region 316 can define a bevel angle L, and contact region 316 and proximal edge 318 can define a bevel angle M. In some cases, bevel angle L can be within a range from about 11.1° to about 15.1°, and bevel angle M can be within a range from about 8.2° to about 12.2°. Optionally, bevel angle L can be about 13.1° and bevel angle M can be about 10.2°.

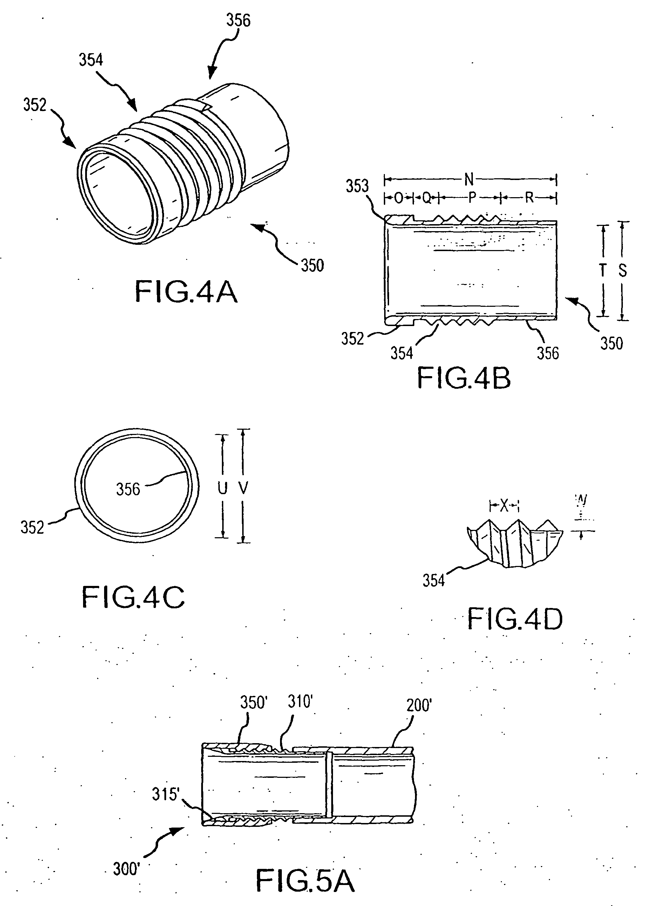

Various features of tip 350 are depicted in Figs. 4A - 4D, as illustrated in Fig. 4A, tip 350 can include a distal tip edge 352, an external thread 354, and a proximal tip edge 356. In some cases, as shown in Fig. 4B, tip 350 can have a length N within a range from about 2.54 mm to about 10.7mm (about 0.10 inch to about 0.50 inch). Optionally, tip 350 can have a length N of about 7.62 mm (about 0.30 inch). In some case, distal tip edge 352 can have a length O within a range from about 0.25 mm to about 2.54 mm (about 0.01 inch to about 0.10 inch). Optionally, distal tip edge 352 can have a length O of about 1.27 mm (about 0.05 inch) . In some embodiments, external thread 354 can have a length P within a range from about 2.03 mm to about 5.33 mm (about 0.08 inch to about 0.21 inch). Optionally, external thread 354 can have a length P of about 3.30 mm (about 0.13 inch). In some embodiments, a distance Q between distal tip edge 352 and external thread 254 can within a range from about 0.25 mm to about 0.76 mm (about 0.01 inch to about 0.03 inch). Optionally, distance Q can be about 0.51 mm (about 0.02 inch). In some cases, proximal tip edge 356 can have a length R within a range from about 1.27 mm to about 3.81 mm (about 0.05 inch to about 3.81 mm). Optionally, proximal tip edge 356 can have a length R of about 2.54 mm (about 0.10 inch). In some embodiments, threaded section 354 can have an major thread diameter S within a range from about 2.41 mm to about 7.49 mm (about 0.095 inch to about 0.295 inch). Optionally, major thread diameter S can be about 4.93 mm (about 0.195 inch). In some embodiments, threaded section 354 can have a minor thread diameter T within a range from about 1.78 mm to about 6.99 mm (about 0.075 inch to about 0.275 inch). Optionally, minor thread diameter T can be about 4.45 mm (about 0.175 inch). In some cases, an outer diameter of proximal tip edge 356 will be approximately equivalent to minor thread diameter T. Typically, distal tip edge 352 includes an end 352 that is blunt or is otherwise configured to be sufficiently smooth so as to not cut tissue when applied thereto under normal operating conditions. As depicted in Fig. 4C, distal tip edge 352 can have an outer diameter U within a range from about 2.29 mm to about 7.52 mm (about 0.090 inch to about 0.296 inch). Optionally, outer diameter U can be about 4.98 mm (about 0.196 inch). In some embodiments, proximal tip edge 356 can have an inner diameter V within a range from about 1.65 mm to about 6.73 mm (about 0.065 inch to about 0.265 inch). Optionally, inner diameter V can be about 4.19 mm (about 0.165 inch). As illustrated in Fig. 4D, threaded section 354 can define a thread depth W and a thread pitch X. In some cases, thread depth W can be within a range from about 0.13 mm to about 0.38 mm (about 0.005 inch to about 0.015 inch). Optionally, thread depth W can be about 1.25 mm (about 0.010 inch). In some cases, thread pitch X can be within a range from 0.25 mm to 0.76 mm (about 0.010 inch to about 0.030 inch). Optionally, thread pitch X can be about 0.51 mm (about 0.020 inch). In some cases, threaded section 354 can have a thread count within a range from about 2.54 threads per mm to about 15.24 threads per mm (about 10 threads per inch to about 60 threads per inch). Optionally, threaded section 354 can have a thread count of about 10.16 threads per mm (about 40 threads per inch).

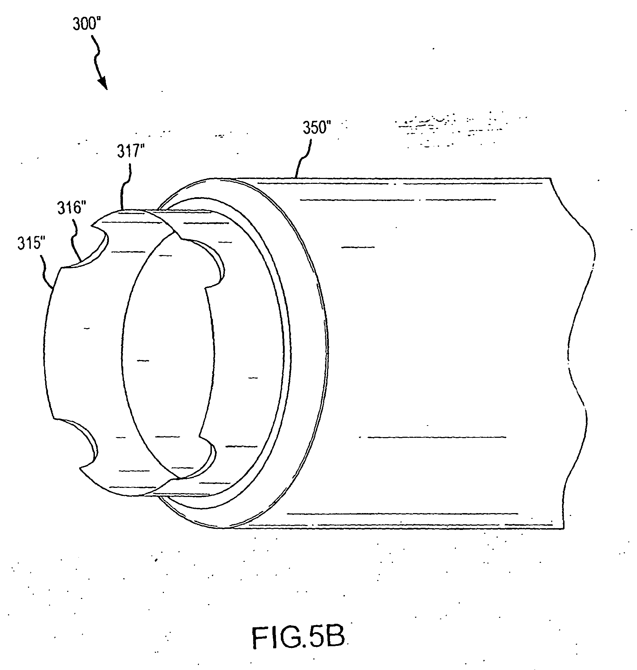

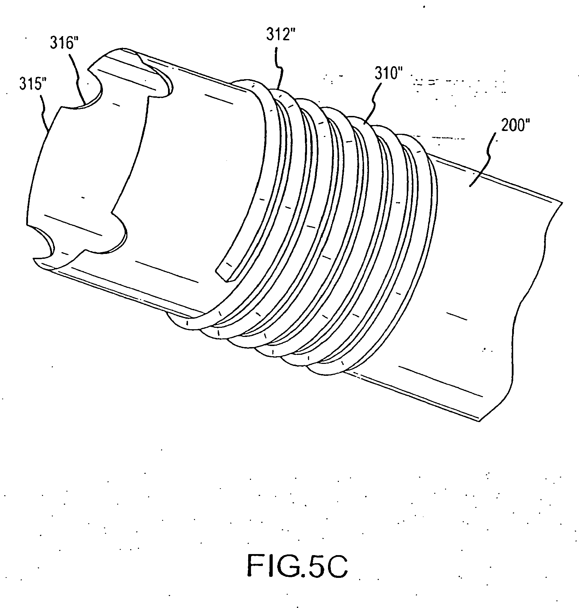

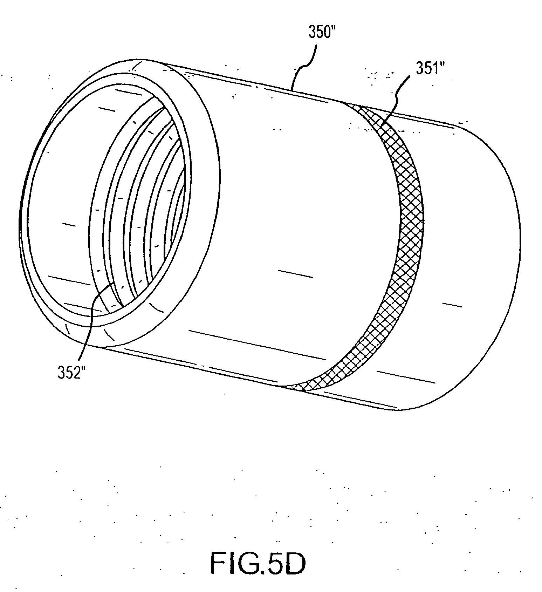

Fig. 5A illustrates a separating assembly 300' according to one embodiment of the present invention, coupled with sheath 200'. Separating 300' includes a separator 310' and a tip 350'. The separating assembly 300' shown in Fig. 5A is similar in some respects to the separating assembly 300 shown in Fig. 2. However, Fig. 5A depicts separator 310' coupled with sheath 200', whereas Fig. 2 depicts tip 350 coupled with sheath 200. Moreover, Fig. 5A shows tip 350' external to separator 310', whereas Fig. 2 shows. separator 310 external to tip 350. In some embodiments, sheath 200' is integral with separator 30', such that separator 310' represents a distal end of sheath 200'. Depending on whether an embodiment includes a separating assembly 300 as shown in FIG. 2 or a separating assembly 300' as shown in FIG. SA, certain features or elements of the respective tips (350, 350) or separator (310, 310') may vary. For example, a separating assembly may include a contact region for contacting a tissue or surface of a patient's body, as further discussed elsewhere herein. This contact region may be disposed on a tip or a separator of a separating assembly. In the embodiment shown in FIG. 2, the contact region is typically disposed on a surface of separator 310. In the embodiment shown in FIG. 5, the contact region is typically disposed on a surface of tip 350'. Separator 310, 310' can include a serrated or angled surface for separating. When in the undeployed configuration, the separator is typically flush with or disposed proximal to the distal end of the tip. When separator is mated with tip in this manner, the separating assembly provides for a smooth tapered, non-separating profile. In some embodiments, an undeployed configuration can refer to a separating assembly having an unexposed separator blade or a non-separating profile. When in the deployed configuration, the separator can be extended distally beyond the distal end of the tip. In some embodiments, a deployed configuration can refer to a separating assembly having an exposed separator blade or a cutting profile. In some deployed configurations, the separator is extended past the tip by about 0.5 mm to about 5 mm. In some embodiments, a separator, which may include a cutter, a stripper, or a dilator, may extend past the tip by about 1.5 mm. In some embodiments, the shape of the separator extending past or configured relative to the tip can vary or be non symmetric around a circumference of the separator. Exemplary separating assembly configurations are described in U.S. Patent Application No: 11/615,005 filed December 22, 2006 (Tissue Separating Systems and Methods) published as US-A-2003/0154296). Relative movement between the tip and separator can be controlled by one or more stops. For example, as shown in FIG. 2, stop 355 can prevent or inhibit separator 310 from extending distally from tip 350 beyond a certain distance. Similarly, as shown in FIG. 5A, stop 315' can prevent or inhibit tip 350' from extending distally from separator 310' beyond a certain distance.

FIGS. 5B-D illustrate a separating assembly 300" according to embodiments of the present invention. Separating assembly 300" includes a separator 310" and a tip 350", and can be coupled with a sheath 200". As shown here, tip 350" is disposed external to separator 310". In some embodiments, sheath 200" is integral with separator 310", such that separator 310" represents a distal end of sheath 200". Tip 350" includes a contact region 351" for contacting a tissue or surface of a patient's body. Contact region 351" may be disposed at any suitable location on tip 350". For example, contact region 351" can be located at a proximal portion of tip 350", a medial portion of tip 350", or at a distal portion, edge, or face of tip 350". Cutter 310" can include separating edge 315" having one or more notches 316" or serrations 317". Separator 310" and tip 350" can be engaged in any of a variety of ways. For example, separator 310" can include threads 312" that interface with threads 352" of tip 350". When in the undeployed configuration, the separator is typically flush with or disposed proximal to the distal end of the tip. When separator is mated with tip in this manner, the separating assembly provides for smooth tapered, non-separating profile. When in the deployed configuration, as shown in FIG. 5B, the separator is typically extended distally beyond the distal end of the tip.

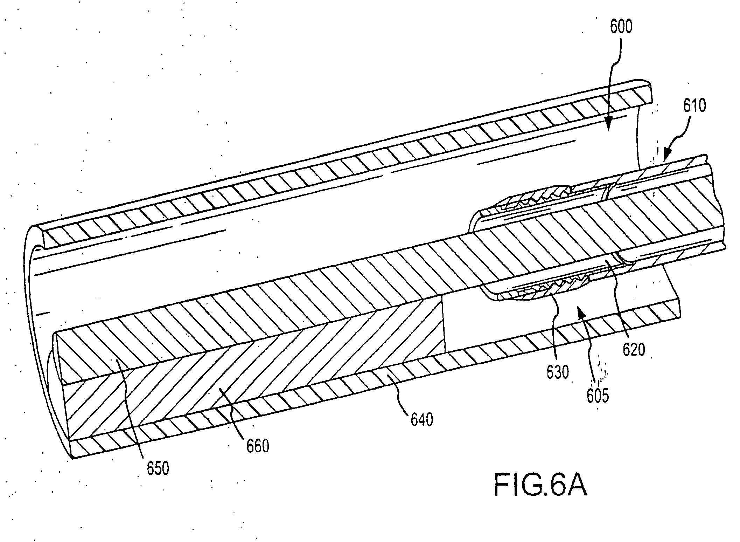

FIGS. 6A-6C illustrate a method of using a cutting assembly according to embodiments of the present invention. As shown in FIG. 6A, cutting system 600 includes a sheath 610 coupled with a tip 620, and a separator 630 engaged with tip 620. Separator 630 and tip 620 may collectively be referred to as cutting assembly 605. A cylindrical portion of separator 630 is disposed in concentric arrangement with a cylindrical portion of tip 620. Sheath 610 can include any of a variety of flexible materials, and typically includes a passage extending therethrough. In some embodiments, sheath 610 is sufficiently flexible to navigate tortuous paths within various body lumens or vessels. Cutting system 600 is advanced along the interior of patient vessel or lumen 640, and over the surface of pacing lead or object 650. As shown in this illustration, separator 630 is retracted from the distal end of tip 620, so as to provide a non-separating profile. It is appreciated that methods may encompass any of a variety of non-separating profiles or undeployed configurations that include an unexposed separating edge, blade, or other suitable separating means or mechanism. As separating system 600 is advanced along the object, the distal end of the system contacts the scar tissue or adhesion 660. In some cases, tissue 660 can be separated from object 650 by forcing the distal end of system 600 against tissue 660, with separating assembly 605 in a non-separating profile.

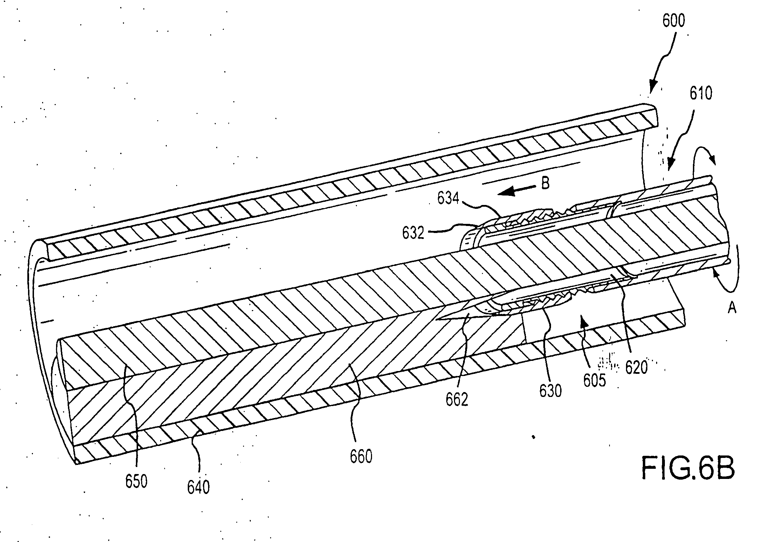

As depicted in FIG. 6B, an operator can rotate sheath 610, which in turn rotates tip 620 relative to separator 630, and thus extends separator 630 distally for cutting tissue 660. In an exemplary embodiment, the operator situates the distal end of separating system 600 so that a contact region 634 of separator 630 touchs a tissue of the patient. Contact region 634 can be disposed at any location on separator 630, and is suitable for contacting a tissue or other component of or within the patient's body. Typically, contact region 634 is disposed along an external surface of separator 630. Depending on the configuration of separating assembly 605, contact region may be disposed on tip 620, as previously discussed in relation to FIGS. 2 and 5A. Friction between contact region 634 and the tissue allows tip 620 to rotate relative to separator 630, and thus the operator can extend, or retract, separator 630 or a portion thereof by rotating sheath 610. The tissue interface surface or contact region 634 can have a finish that facilitates frictional resistance. For the example, contact region 634 may include a sand blasted, grooved, coated, holed, pitted, notched, or knurled surface. The contact region surface can be pressed against the tissue surface so as to anchor or fix the contact region relative to the tissue surface or site.

Although this deployment procedure is described in terms of frictionally engaging a tissue surface with a contact region, it is understood that in some cases other mechanisms or methods can provide for relative rotational movement between the separator and tip, so as to allow the separating assembly to switch or transform between a deployed configuration and an undeployed configuration. For example, suitable combinations of knobs, gears, triggers, interior bolts, and other activating mechanisms or means can be used to cause the separator to rotate relative to the tip. It is appreciated that methods may encompass any of a variety of separating profiles or deployed configurations that include an exposed separating means or mechanism such as an edge or a blade. In some cases, the separating assembly is adapted to switch between a deployed configuration where a portion of the separating means is exposed and an undeployed configuration where the portion of the separating mechanism or means is unexposed. The portion of the separating mechanism or means may include all or some of the separating mechanism or means. Some embodiments may include temporarily fixing or releasably engaging a portion of a separating assembly with a site adjacent to or on the tissue which is to be separated. For example, a separator or tip can be temporarily fixed with a site via retractable spikes or projections disposed on an external surface of the cutter or tip. Similarly, a separator or tip can include a surface having directional projections, such as microprojections, that lay flat when the separator or tip is rotated in one direction, and stand up straight or otherwise deploy when the separator or tip is rotated in the other direction. In some cases, such projections provide a sufficiently soft and atraumatic surface so as to not cause undesired tissue damage when the device is, rotated during separating operations.

As shown in FIG. 6B, the operator can rotate sheath 610 as indicated by arrow A, so as to extend or deploy separator 630 distally beyond tip 620, thus providing a separating profile for separating system 600. Separating assembly 605 can be advanced within lumen 640 as indicated by arrow B, such that distal separating edge 632 is applied to tissue 660, and makes a separation, cut, or incision 662 in tissue 660. In some embodiments, tissue 660 includes scar tissue or adhesions. In some embodiments, distal separating edge 632 is applied to tissue 660 so as to separate tissue 660 from object or lead 650 or from other tissue. In some embodiments, distal separating mechanism or edge 632 is applied to tissue 660 so as to strip tissue 660 from object or lead 650 or from other tissue. In some embodiments, distal separating edge 632 is applied to tissue 660 so as to dilate tissue 660 away from object or lead 650 or from other tissue. It is understood that in some embodiments, the distal extension of separator 630 involves rotating tip 620 in a counter-clockwise direction relative to separator 630. In other embodiments, the distal extension of cutter 630 involves rotating tip 620 in a clockwise direction relative to separator 630, depending on the orientation of the threaded arrangement between tip 620 and separator 630. The threaded interface between separator 630 and tip 620 can be designed to minimize friction and to keep out blood or tissue. The threaded mating surfaces may be polished or coated and matched to fine tolerances or other methods may be employed such as seals or greases. The pitch or count of the threads can be made to provide a known extension or retraction distance within a certain number of rotations or fractions thereof. This can allow an operator to carefully calibrate then extent to which a separating edge is deployed or undeployed via actuation of the threadable coupling between the tip and the separator.

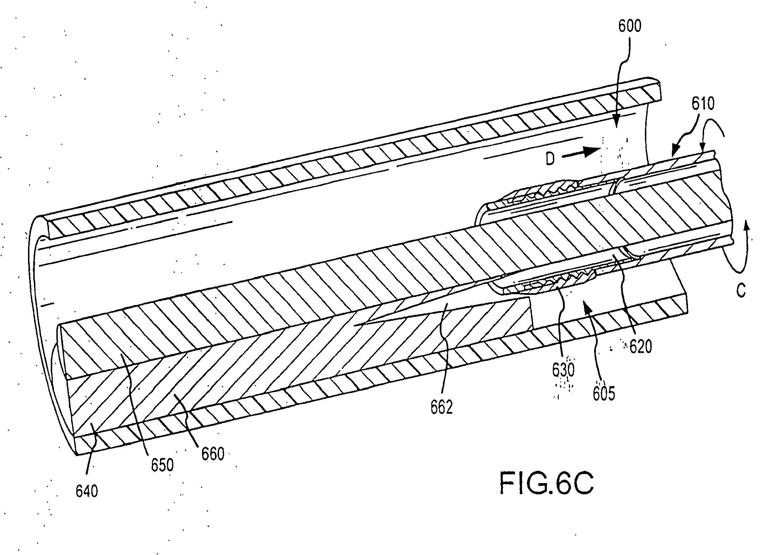

As depicted in FIG. 6C, an operator can rotate sheath 610, which in turn rotates tip 620 relative to separator 630, and thus retracts separator 630 proximally. For example, the operator can rotate sheath 610 as indicated by arrow C, so as to retract or dispose separator 630 flush with or proximal to a distal end of tip 620, thus providing a non-separating profile for separating system 600. Accordingly, methods may include rotating the sheath to induce relative rotational movement between the separator and the tip so as to undeploy the separating means of the separator. Similarly, methods may include rotating the sheath so as to undeploy the separating assembly. Separating assembly 605 can be retracted within lumpen 640 as indicated by arrow D, such that separating assembly 605 is removed from or moved proximal relative to cut or incision 662 in tissue 660. It is understood that in some embodiments, the proximal retraction of separator 630 involves rotating tip 620 in a counter-clockwise direction relative to separator 630. In other embodiments, the proximal retraction of separator 630 involves rotating tip 620 in a clockwise direction relative to separator 630, depending on the orientation of the threaded arrangement between tip 620 and separator 630.

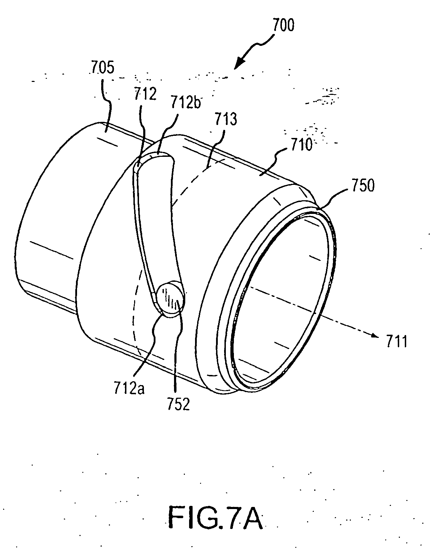

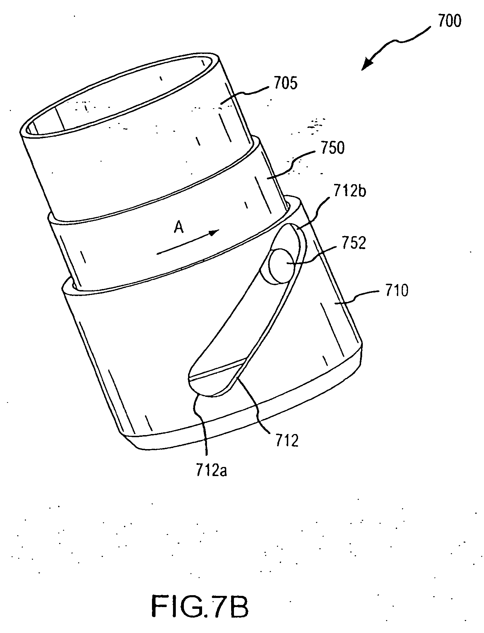

FIGS. 7A and 7B depict a separating assembly 700 according to embodiments of the present invention. Separating assembly 700 can include a separator 710 in operative association with a tip 750. Here, separator 710 includes a slot 712, and tip 750 includes a key 752. Slot 712 includes a first end 712a disposed toward a distal portion of cutter 710, and a second end 712b disposed proximal to first end 712a. Key 752 cooperatively engages slot 712, and slot 712 is aligned at an angle offset from a central longitudinal axis 711 or a circumferential band 713 of separator 710, such that when cutter 710 is rotated relative to tip 750 in one direction, key 752 is disposed more closely to first end 712a of the slot, as shown in FIG. 7A. Conversely, when separator 710 is rotated relative to tip 750 in the opposite direction, key 752 is disposed more closely to second end 712b of the slot, as shown in FIG. 7B. In some embodiments, the offset angle can be selected or calibrated to allow a user to carefully adjust the distance which the separator is extended or retracted, by correlating a movement in linear distance with a rotational movement of the sheath. Any of a variety of slot and key configurations or connections are contemplated. In some cases, the separator includes a key, and the tip includes a slot. A separating assembly may include a tip disposed at least partially within a separator, or a separator disposed at least partially within a tip. In either case, the tip may include a key, a slot, or both, and the separator may include a slot, a key, or both. In some embodiments, sheath 705 is integral with tip 750, such that tip 750 represents a distal end of sheath 705. In some embodiments, a distal end of sheath 705 is fixed with tip 750. When initially placed within a vessel or lumen of a patient, separating assembly 700 is typically disposed in an undeployed configuration, as shown in FIG. 7A, whereby a distal end of separator 710 is flush with or proximal to a distal end of tip 750. Once the assembly 700 is positioned at or near a tissue which an operator wishes to separate, the operator can maneuver the assembly 700 so that an external surface of separator 710 frictionally contacts a tissue surface of the patient. The operator can then rotate shaft 705 so that tip 750 rotates relative to separator 710 as shown by arrow A, thus extending a portion of separator 710 distally beyond tip 750, as shown in FIG. 7B. The separating assembly 700 is thus in a deployed configuration and ready for separating the desired tissue.

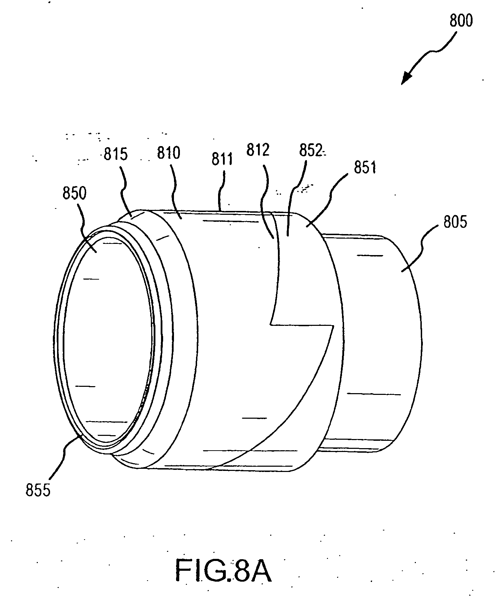

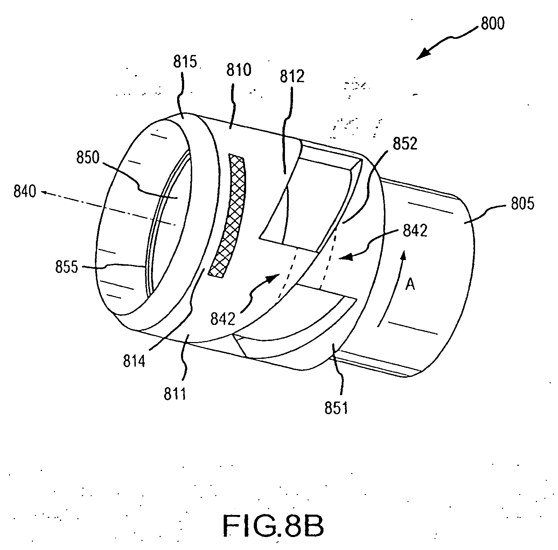

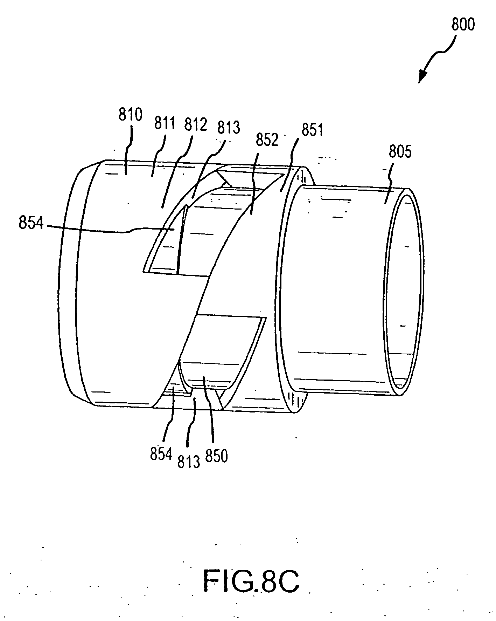

FIGS. 8A-8C depict a separating assembly 800 according to embodiments of the present invention. Separating assembly 800 can include a separator 810 in operative association with a tip 850. For example, separator 810 can be coupled with tip 850 via a cam connection. In some cases, separator 810 includes distal cam 811 having a distal cam interface 812, and tip 850 or sheath 805 includes a proximal cam 851 having a proximal cam interface 852. Distal cam interface 812 and proximal cam interface 852 are cooperatively engaged such that relative rotational movement between distal cam 811 and proximal cam 851 is associated with relative linear movement between distal cam 811 and proximal cam 851. In this way, relative rotation of the cams 811, 851 can cause the separator 810 to extend and retract relative to tip 850. In some configurations, the cam interfaces provide an angled profile. Optionally, the cam interfaces can provide a curved or eccentric profile. An angled or curved profile can be designed or calibrated to allow a user to carefully adjust the distance which the separator is extended or retracted, by correlating a movement in linear distance with a rotational movement of the sheath. Thus, an operator can control the extent to which a separating edge or means is deployed or undeployed via actuation of the cam coupling between the tip and the separator. FIG. 8A depicts separating assembly in an undeployed configuration, wherein a separating edge or means 815 of separator 810 is disposed flush with or proximal to a distal edge 855 of tip 850. In use, as shown in FIG. 8B, a contact region 814 of separator 810 can engage a body tissue or other surface or object within a patient, and the operator can rotate tip 850 relative to separator 810 as indicated by arrow A, so as to provide linear separation between distal cam 811 and proximal cam 852 as distal cam interface 812 and proximal cam interface 852 slide against each other. Distal cam interface 812 and proximal cam interface can be aligned at an angle offset from a central longitudinal axis 840 or a circumferential band 842 of distal cam 811 or proximal cam 851, such that when separator 810 is rotated relative to tip 850, relative linear motion between separator 810 and tip 850 is induced. In some embodiments, sheath 805 is integral with tip 850, such that trip 850 represents a distal end of sheath 805. In some embodiments, a distal end of sheath 805 is fixed with tip 850. As shown in FIG. 8B, rotational- actuation of cam interfaces 812 and 852 disposes separating assembly 800 in a deployed configuration, ready for separating the desired tissue. FIG. 8C also depicts separating assembly 800 in a deployed configuration. As seen from this perspective, tip 850 can include a stop 854 configured to engage an edge or projection 813 of separator 810. Stoup 854 can prevent or inhibit separator 810 from excessive or unwanted movement in the distal direction. In some cases, stop 854 prevents or inhibits separator 810 from becoming detached or disengaged from tip 850 or sheath 805.

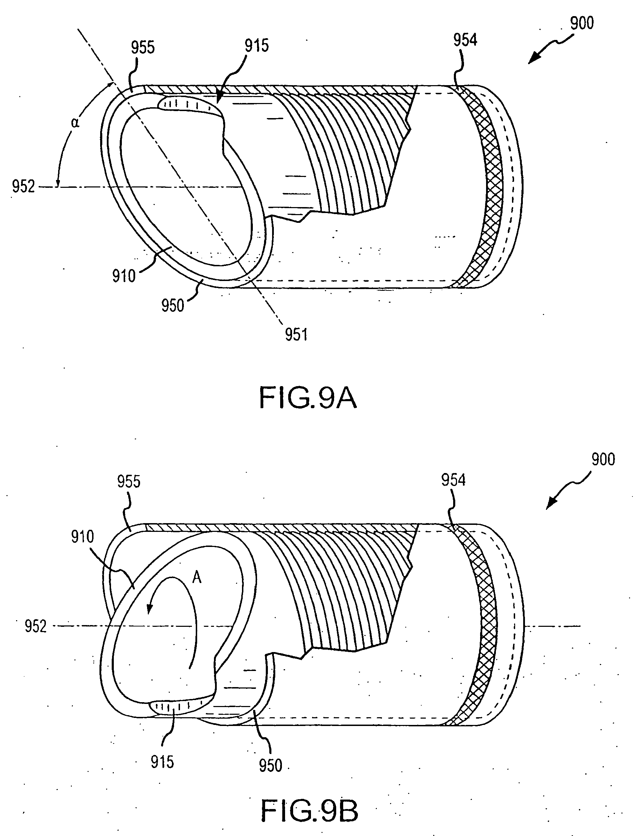

FIGS. 9A and 9B show a separating assembly 900 according to embodiments of the present invention. Separating assembly 900 can include a separator 910 in operative association with a tip 950. For example, separator 910 can be coupled with tip 950 via a threaded connection. Relative rotation between separator 910 and tip 950 can be the result of or the cause of relative positioning between separator 910 and tip 950 along a common longitudinal axis. FIG. 9A depicts separating assembly 900 in an undeployed configuration, wherein a separating edge or blade 915 of separator 910 is disposed flush with or proximal to a distal edge 955 of tip 950. A distal end or face of tip 950 is beveled so as to define a plane 951 that intersects a central longitudinal axis 952 of tip 950 at an angle α, which in some cases can be within a range from about 10 degrees to about 80 degrees. A distal end or face of separator 910 may present a similar beveled configuration. In use, a contact region 954 of tip 950 can engage a body tissue or other surface or object within a patient, and the operator can induce relative rotational movement between tip 950 and separator 910. As shown in FIG. 9B, the operator can rotate separator 910 relative to tip 950 as indicated by arrow A, so as to expose separating blade 915. In some embodiments, separating blade 915 is exposed and configured for use, and separating assembly 900 is in a deployed configuration, while separating blade 915 remains proximal to distal edge 955 of tip 950.

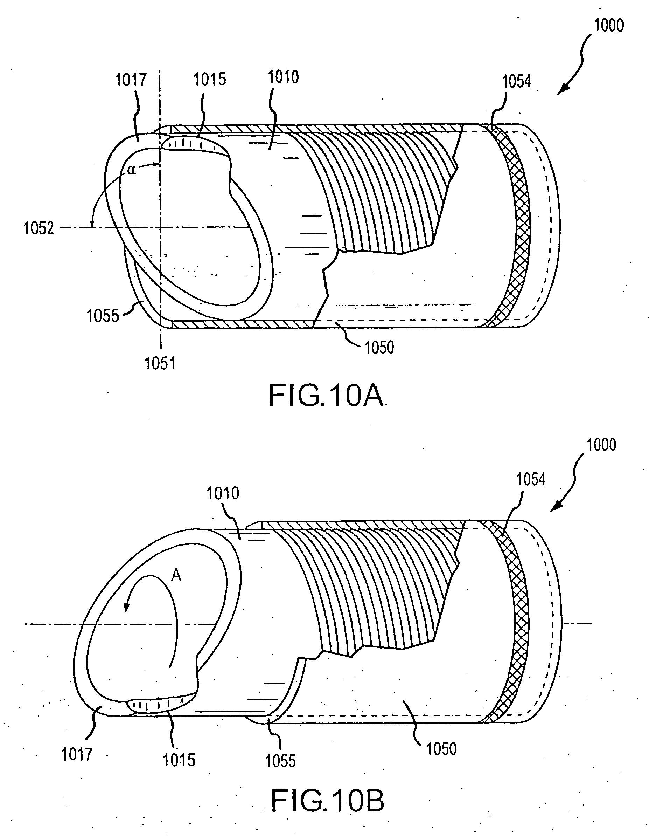

FIGS. 10A and 10B show a separating assembly 1000 according to embodiments of the present invention. Separating assembly 1000 can include a separator 1010 in operative association with a tip 1050. For example, separator 1010 can be coupled with tip 1050 via a threaded connection. Relative rotation between separator 1010 and tip 1050 can be the result of or the cause of relative positioning between separator 1010 and tip 1050 along a common longitudinal axis. FIG. 10A depicts separating assembly 1000 ins an undeployed configuration, wherein a separating edge or blade 1015 of separator 1010 is disposed flush with or proximal to a distal edge 1055 of tip 1050. In some cases, a distal end or portion 101 of separator 1010 can be positioned distal to distal tip edge 1055, when separating assembly is in the undeployed configuration and separating blade or means 1015 is unexposed. A distal end or face of tip 1050 defines a plane 1051 that perpendicularly intersects a central longitudinal axis 1052 of tip 1050. In some cases, plane 105 intersects axis 1052 at an angle α, which in some cases can be within a range from about 75 degrees to about 90 degrees. A distal end or face of separator 1010 may present a beveled configuration as described above with reference to FIGS. 9A and 9B. In use, a contact region 1054 of tip 1050 can engage a body tissue or other surface or object within a patient, and the operator can induce relative rotational movement between tip 1050 and separator 1010. As shown in FIG. 10B, the operator can rotate separator 1010 relative to tip 1050 as indicated by arrow A, so as to expose separating blade 1015, and thus place separating assembly 1000 in a deployed configuration.

In addition to being well suited for the removal or detachment of pacing leads from a patient, embodiments of the present invention are well suited for detaching or removing any of a variety of objects from a patient, such as catheters, wires, implants, or other foreign bodies, and for separating tissue from other neighboring or adjacent tissue. Such objects may be disposed in veins, arteries, or any body lumen, cavity, or tissue.

Embodiments of the invention have now been described in detail. However, it will be appreciated that the invention may be carried out in ways other than those illustrated in the aforesaid discussion, and that certain changes and modifications may be practiced within the scope of the appended claims.

## Claims

1. A separating system for detaching a pacing lead from a patient, comprising:
a sheath (200) having a distal end; and
a separating assembly coupled with the distal end of the sheath, the separating assembly comprising a tip (350) and a separator (310) the separator having a separating mechanism;
wherein the sheath (200) is fixed with either the tip (350) or the separator (310), the separating mechanism comprising a circular distal separating edge (312), the tip comprising a blunt distal end, and each of the sheath (200), the tip (350), and the separator (310) being configured for placement over the pacing lead, the tip and the separator being configured for relative rotational movement;
**characterized in that**:
the separating assembly is adapted to switch upon the relative rotational movement of the tip and the separator (310) between a deployed configuration where the circular distal separating edge (312) of the separating mechanism is exposed and an undeployed configuration where the circular distal separating edge (312) of the separating mechanism is unexposed.

2. The separating system according to claim 1, wherein the separator (310) and the tip (350) are coupled via a threaded, a slot and key, or a cam connection.

3. The separating system according to claim 1, further comprising a contact region (316), wherein the contact region (316) is either disposed on the separator (310) and adapted to releasably fix the separator (310) relative to the patient, or disposed on the tip (350) and adapted to releasably fix the tip (350) relative to the patient.

4. The separating system according to claim 3, wherein the contact region (316) comprises a member selected from the group consisting of a sand blasted surface, a grooved surface, a coated surface, a knurled surface, a holed surface, a pitted surface, and a notched surface.

5. The separating system according to claim 1, wherein in the deployed configuration the separating mechanism extends past the tip (350) a distance in the range from about 0.5 mm to about 3 mm.

6. The separating system according to claim 1, wherein the separating assembly comprises a member selected from the group consisting of a cutting assembly, a dilating assembly, and a stripping assembly.

7. The separating system according to claim 1, wherein the separator (310) comprises a member selected from the group consisting of a cutter, a dilator, and a stripper.

8. The separating system according to claim 1, wherein the separating mechanism comprises a member selected from the group consisting of a cutting mechanism, a dilating mechanism, and a stripping mechanism.

9. The separating system according to claim 1, wherein the separating mechanism comprises a blade.

## Patentansprüche

1. Trennsystem zum Lösen eines Schrittmacherdrahts von einem Patienten, wobei das Trennsystem Folgendes umfasst:
einen Schaft (200) mit einem distalen Ende und
eine Trennanordnung, die mit dem distalen Ende des Schafts verbunden ist, wobei die Trennanordnung eine Spitze (350) und eine Trennvorrichtung (310) umfasst, wobei die Trennvorrichtung einen Trennmechanismus aufweist;
wobei der Schaft (200) in Bezug auf die Spitze (350) oder die Trennvorrichtung (310) fixiert ist, wobei der Trennmechanismus eine kreisförmige, distale Trennschneide (312) umfasst, wobei die Spitze ein stumpfes distales Ende umfasst, und der Schaft (200), die Spitze (350) und die Trennvorrichtung (310) ausgebildet sind, um über dem Schrittmacherdraht angeordnet zu werden, wobei die Spitze und die Trennvorrichtung für eine relative Rotationsbewegung ausgebildet sind,
**dadurch gekennzeichnet, dass**
die Trennanordnung geeignet ist, um bei relativer Rotationsbewegung der Spitze und der Trennvorrichtung (310) zwischen einer einsatzbereiten Position, in der die kreisförmige, distale Trennschneide (312) des Trennmechanismus frei liegt, und einer nicht einsatzbereiten Position, in der die kreisförmige, distale Trennschneide (312) des Trennmechanismus nicht frei liegt, umzuschalten.

2. Trennsystem nach Anspruch 1, worin die Trennvorrichtung (310) und die Spitze (350) über eine Gewinde-, eine Nut-und-Feder- oder eine Nockenverbindung verbunden sind.

3. Trennsystem nach Anspruch 1, das ferner einen Kontaktbereich (316) umfasst, wobei der Kontaktbereich (316) entweder auf der Trennvorrichtung (310) angeordnet ist und geeignet ist, um die Trennvorrichtung (310) in Bezug auf den Patienten lösbar zu fixieren, oder auf der Spitze (350) angeordnet ist, um die Spitze (350) in Bezug auf den Patienten lösbar zu fixieren.

4. Trennsystem nach Anspruch 3, worin der Kontaktbereich (316) ein Element umfasst, das aus der aus folgenden bestehenden Gruppe ausgewählt ist: einer sandgestrahlten Oberfläche, einer gerillten Oberfläche, einer beschichteten Oberfläche, einer gerändelten Oberfläche, einer gelöcherten Oberfläche, einer Oberfläche mit Unebenheiten und einer gekerbten Oberfläche.

5. Trennsystem nach Anspruch 1, worin der Trennmechanismus sich in der einsatzbereiten Position etwa 0,5 bis etwa 3 mm über die Spitze (350) hinaus erstreckt.

6. Trennsystem nach Anspruch 1, worin die Trennanordnung ein Element umfasst, das aus der aus einer Schnittanordnung, einer Dilatationsanordnung und einer Ablöseanordnung bestehenden Gruppe ausgewählt ist.

7. Trennsystem nach Anspruch 1, worin die Trennvorrichtung (310) ein Element umfasst, das aus der aus einer Schneidvorrichtung, einer Dilatationsanordnung und einer Ablösevorrichtung bestehenden Gruppe ausgewählt ist.

8. Trennsystem nach Anspruch 1, worin der Trennmechanismus ein aus der aus einem Schneidmechanismus, einem Dilatationsmechanismus und einem Ablösemechanismus bestehenden Gruppe ausgewähltes Element umfasst.

9. Trennsystem nach Anspruch 1, worin der Trennmechanismus eine Klinge umfasst.

## Revendications

1. Système de séparation pour détacher une électrode de stimulation d'un patient, comprenant:
une gaine (200) ayant une extrémité distale; et
un ensemble de séparation couplé à l'extrémité distale de la gaine, l'ensemble de séparation comprenant une pointe (350) et un séparateur (310), le séparateur comportant un mécanisme de séparation;
où la gaine (200) est fixée soit à la pointe (350) soit au séparateur (310), le mécanisme de séparation comprenant un bord de séparation circulaire distal (312), la pointe comprenant une extrémité distale émoussée, et chacun parmi la gaine (200), la pointe (350) et le séparateur (310) étant configuré pour être placé sur l'électrode de stimulation, la pointe et le séparateur étant configurés en vue d'un mouvement de rotation relatif;
**caractérisé en ce que**:
l'ensemble de séparation est apte à être commuté lors d'un mouvement de rotation relatif de la pointe et du séparateur (310) entre une configuration déployée dans laquelle le bord de séparation circulaire distal (312) du mécanisme de séparation est exposé, et une configuration non déployée où le bord de séparation circulaire distal (312) du mécanisme de séparation n'est pas exposé.

2. Système de séparation selon la revendication 1, dans lequel le séparateur (310) et la pointe (350) sont couplés par une connection à filetage, une fente et clé ou une connection à came.

3. Système de séparation selon la revendication 1, comprenant en outre une région de contact (316), dans lequel la région de contact (316) est soit disposée sur le séparateur (310) et est apte à fixer relâchablement le séparateur (310) relativement au patient, soit elle est disposée sur la pointe (350) et est apte à fixer relâchablement la pointe (350) relativement au patient.

4. Système de séparation selon la revendication 3, dans lequel la région de contact (316) comprend un élément sélectionné dans le groupe consistant en surface sablée, surface rainurée, surface revêtue, surface molletée, surface creusée, surface piquée et surface encochée.

5. Système de séparation selon la revendication 1, dans lequel dans la configuration déployée, le mécanisme de séparation s'étend au-delà de la pointe (350) selon une distance dans la plage d'environ 0,5 mm à environ 3 mm.

6. Système de séparation selon la revendication 1, dans lequel l'ensemble de séparation comprend un élément sélectionné dans le groupe consistant en un ensemble de coupe, un ensemble de dilatation et un ensemble de dévêtissage.

7. Système de séparation selon la revendication 1, dans lequel le séparateur (310) comprend un élément sélectionné dans le groupe consistant en un organe de coupe, un dilatateur et un dévêtisseur.

8. Système de séparation selon la revendication 1, dans lequel le mécanisme de séparation comprend un élément sélectionné dans le groupe consistant en un mécanisme de coupe, un mécanisme de dilatation et un mécanisme de dévêtissement.

9. Système de séparation selon la revendication 1, dans lequel le mécanisme de séparation comprend une lame.
